(19) Europäisches Patentamt
European Patent Office
Office européen des brevets

(11) Publication number: 0 225 648
A2

(12) # EUROPEAN PATENT APPLICATION

(21) Application number: 86117266.6

(22) Date of filing: 11.12.86

(51) Int. Cl.[4]: A 61 K 39/39

(30) Priority: 13.12.85 IL 77323

(43) Date of publication of application: 16.06.87
Bulletin 87/25

(84) Designated Contracting States: AT BE CH DE ES FR GB GR IT LI LU NL SE

(71) Applicant: YEDA RESEARCH AND DEVELOPMENT COMPANY, LTD., Weizmann Institute of Science Herzl Street at Yavne Road P.O. Box 95, Rehovot 76100 (IL)

(72) Inventor: Feldman, Michael, Meonot Wix Weizmann Inst.of Science, Rehovot (IL)
Inventor: Tzehoval, Esther, 125 Jabotinsky Street, Ramat Gan (IL)
Inventor: Fridkin, Matityahu, 23 Miller Street, Rehovot (IL)
Inventor: Dagan, Shlomo, Bustani Street 12, Rehovot (IL)

(74) Representative: Vossius & Partner, Siebertstrasse 4 P.O. Box 86 07 67, D-8000 München 86 (DE)

(54) **Compositions of matter having highly potentiated antigenic activity.**

(57) Described are novel compositions of matter which are potent immunogens and can be applied to mammals in order to protect same against viral, bacterial and parasitic infections. The potent immunogens are conjugates of the tetrapeptide tuftsin or of an active analog of tuftsin or of a short peptide containing the tuftsin sequence or a short peptide containing the sequence of an active analog of tuftsin with a wide variety of entities having antigenic effect.

EP 0 225 648 A2

Our ref.: W 075 EP
Case: T/611
Yeda Research and Development Company Limited
Rehovot 76100, Israel

December 11, 1986

VOSSIUS + PARTNER
PATENTANWÄLTE
8000 MÜNCHEN 86
SIEBERTSTRASSE 4
TELEFON 474075

# COMPOSITIONS OF MATTER HAVING HIGHLY POTENTIATED ANTIGENIC ACTIVITY

## FIELD OF THE INVENTION:

The invention relates to compositions of matter for use as vaccine or for similar purposes, in human and veterinary medicine. The compositions of matter comprise the tetrapeptide tuftsin (Thr-Lys-Pro-Arg), the analog of tuftsin $[Ala]^1$-tuftsin, the pentapeptide tuftsin-Gly, or other active short peptides containing the Pro-Arg entity of tuftsin, which have a similar effect, conjugated with an entity adapted to result in an immunogenic response of the mammal. The immunogenic effect of such antigens is substantially potentiated when used as conjugate with tuftsin or an analog thereof. The tuftsin entity is advantageously covalently bonded to the antigenic moiety, but other forms of strong bond between the two entities may be resorted to.

## BACKGROUND OF THE INVENTION:

When a foreign pathogen penetrates a living mammalian organism, it is first taken up, i.e., phagocytosed, by cells of the macrophage category. These may include polymorphonulcears, monocytes and macrophages. It has long been known that immunoglobulins exert a so-called opsonizing, i.e., activating effect on the phagocytic function of macrophages. Using phosphocellulose separation of immunoglobulins, V.Najjar demonstrated some 12 years ago that there is one particular fraction of immunoglobulin which stimulates phagocytosis. He subsequently discovered that this stimulation is not mediated via the intact immunoglobulin molecule. Rather a tetrapeptide composed of Thr-Lys-Pro-Arg is split off the Fc portion of the immunoglobulin heavy chain and this tetrapeptide, which was termed tuftsin, actually triggers phagocytosis. It appears that the tetrapeptide is cleaved of the $CH_2$ portion of the immunoglobulin via 2 distinct steps: the first step seems to be mediated via a splenic

enzyme which splits the carboxy end at the Arg, and the second step, which takes place on the surface of the polymorphonuclear cells or macrophages, is the splitting of the amino end of Thr by leukokininase. Extensive studies were carried out in which the synthetic tuftsin was shown to exert a significant effect on phagocytosis by macrophages. Macrophages manifest an additional very important function: a defined subset of macrophages constitutes the cells which present antigens to lymphocytes and this triggers the immunogenic process. Tuftsin was demonstrated by us to augment significantly the immunogenic capacity of the macrophage.

SUMMARY OF THE INVENTION:

There are provided compositions of matter which are characterized by a very potent immunogenic effect on mammals. Such compositions can be used for a variety of purposes, the main ones being the use as effective vaccines adapted to protect mammals (including humans) against viral, bacterial and parasitic infections.

The novel compositions of matter comprising of the tetrapeptide tuftsin or of an active analog of tuftsin or of a short peptide containing the sequence of tuftsin or of a short peptide containing the sequence of an active analog of tuftsin conjugated with an entity adapted to elicit an immunogenic response.

The dominant sequence in the tuftsin molecule seems to be the Pro-Arg moiety; see Tzehoval et al., Proc. Nat. Acad. Sci. USA 75, 3400 (1978), although the entire tetrapeptide is recognized by the tuftsin receptor. Based on this, it is possible to prepare certain analogs of tuftsin which have analogous, and even better properties than natural tuftsin: each of these has to be experimentally evaluated for its specific characteristics, which can be done without difficulty by

conventional techniques. Based on this, it is clear that the present invention encompasses the use of conjugates either with tuftsin, or with pentapeptides which contain the tuftin sequence or with effective analogs of tuftsin.

As to the preparation of effective vaccines, it is possible to attach tuftsin to a wide variety of substances. Amongst these there may be mentioned various types of sugars, oligosugars, nucleic acids, cell surface constituents, membrane substances, vitamins, polypeptides, peptide hormones, hormones, and a variety of polymeric substances. It can also be attached to a variety of organic or inorganic materials (silica, glass etc.). Within the scope of the invention are envisaged conjugates of tuftsin with any suitable type of antigenic moiety.

The immunogenic effect of such entities is highly potentiated when conjugated to tuftsin or to an analog thereof. As will be explained in greater detail hereinafter, the simultaneous application of tuftsin and of an antigen does not result in vivo in such potentiated effect. Thus the effect of tuftsin-antigenic moiety conjugates can be termed an effect of potentiation.

The present invention is based on studies started a few years ago, when we investigated whether and how tuftsin activates the immunogenic function of macrophages.

For this purpose we developed an in vitro antigen-specific "education" system which is strictly macrophage-dependent and amenable to experimental manipulation: monolayers of macrophages are fed with antigen, then spleen cells are seeded on these macrophage-monolayers and cultured for 18 h. The nonadherent lymphocytes are then collected, irradiated and injected intrafootpad into syngeneic mice. These migrate to the regional lymph nodes where they recruit antigen-specific

T lymphocytes. Hence the regional popliteal lymph nodes are excised 6 days later, and their cells tested for the specific response to the original test antigen. Using this system, we discovered that when the antigen is fed to macrophages simultaneously with tuftsin, the immune response ( as measured by antigen-specific proliferation of the lymph node cells) is significantly higher than when the macrophages are pulsed with only the antigen. Experimental analysis indicated that tuftsin activates the immunogenic function of the antigen-presenting macrophage. Contrary to what may be expected as the basis of this, in vivo, simultaneous injection into mice of antigen (we used the protein antigens keyhole limpet hemocyanin (KLH) and bovine serum albumin (BSA). as models) plus tuftsin does not result in such activation. Under in vivo conditions, the probability that the antigen and tuftsin will bind to the same macrophage at optimal concentration is very low. Thus in vitro results differ from those in vivo.

According to the invention, in order to have the tuftsin arrive at the desired location and concentration together with the anitgenic moiety, the two were covalently attached to each other, forming a stable entity. The covalently bonded tuftsin-antigen entity was used in order to evaluate the immunization resulting in experimental animals. A variety of proteins was used as antigen, and it was found that when such antigen was injected simultaneously with tuftsin, and the results were compared with those obtained by a corresponding antigen-tuftsin conjugate, the immune response attained with the conjugate was substantially higher than that with these applied simultaneously.

It is stressed that classical adjuvants, such as complete Freund's adjuvant, and also the well-defined muramyl dipeptide adjuvant, have severe side-effects and thus they cannot be used in humans. The

natural tetrapeptide tuftsin, and analogs of same, which have the sequence of natural peptides, do not exhibit such deleterious effects, and thus they can be conjugated with weak immunogens, in order to highly potentiate the resulting immune response obtained from the application to mammals of such conjugates. Analogs of tuftsin, such as the tetrapeptide $[Ala]^1$-tusftsin and the pentapeptide tuftsin-Gly, are effective components of conjugates of the invention, which have an equivalent, or even better effect than that of tuftsin. Whenever the term "tuftsin" is used herein, it is intended to include also such analogs.

Conjugates of tuftsin with suitable viral, bacterial and other pathogenic antigens are within the scope of the invention, providing effective vaccines against a variety of pathogens.

The following Examples are intended to illustrate the principles and applications of the present invention. They are to be construed in a demonstrative and non-limitative manner.

EXAMPLES:

Prepartion of Tuftsin-Protein Conjugates

1. BSA-tuftsin-OH conjugates (via N-terminal)

p-Aminophenylacetyl tuftsin (30 mg; 40 μmol) was dissolved in cold 2N HCl (0.4 ml) and diazotized by addition of ice-cold aqueous sodium nitrite (0.1 M; 0.4 ml). After standing at 4°C for 7 min the reaction mixture was combined with a solution of BSA (68 mg) in $NaHCO_3$ (0.5 M; 4.0 ml), and the pH was adjusted to 8.5 with concentrated aqueous $Na_2CO_3$. The colorless solution immediatly turned brown-red. After reacting for 10 h at 4°C, the mixture was dialyzed against distilled water and then lyophilized, yielding a brown powder of BSA-tuftsin (32 mg). Amino acid analysis revealed that an average of 36 tuftsin moieties were bound to each molecule of BSA. Changing of tuftsin-derivative/BSA ratio will affect loading of the protein. Preparations having 8-36 molecules of tuftsin/BSA were obtained.

2. H-BSA-tuftsin conjugates (via C-terminal)

These were similarly obtained, using

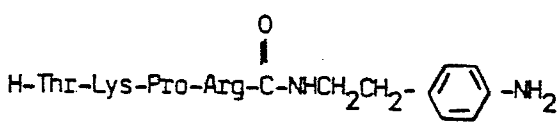

3. Binding of peptide to protein

This was achieved via attachment to tyrosine, histidine and other amino acid residues. Binding can also be achieved amongst others, through formation of amide bonds, i.e., coupling on amino or carboxyl residues of the peptide to the corresponding residues of the proteins. All the reagents known to form amide bonds can, in principle, be used

for this purpose. Coupling of peptide to protein can also be achieved by attaching an "active arm" to the peptide which will react with a corresponding "active arm" on the polymer, as can be generally described;

Tuftsin - A* + protein - B*      Tuftsin-A-B-protein

for example A = SH-carrying molecule (such as cystein)

B = a maleiimide 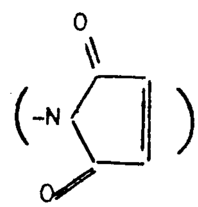 carrying molecule.

4. Proteins

Tuftsin-protein conjugates were prepared in our laboratory, using ribonuclease, KLH, and BSA as the protein carriers. Any protein can be used for this purpose.

Immunogenic effect of antigen-tuftsin conjugates

We first studied the immunogenic effect in vitro of two protein antigens, when these were fed to macrophages in culture. Macrophages were pulsed with antigen then overlaid with spleen lymphocytes. The latter, following 18 h of sensitization were injected into mice, and the recruited effector T-cells were tested for antigen-specific T-cell proliferation 6 days later. BSA alone was found not to elicit any response, whereas both BSA-tuftsin-OH and BSA + H-tuftsin-OH evoked a high level of immunity. H-tuftsin-BSA was similar to BSA in its weak immunogenic capacity. The conjugates of BSA-Tuftsin-OH were very potent immunogens.

Similar tests were carried out using KLH as an antigen, and the results were essentially similar. It should, however, be added that the KLH, being a more potent immunogen, evoked immune responses when the macrophages were pulsed with the antigen and the lymphocytes were assayed for lymphoproliferation at doses which were lower than those of BSA by two logs.

We then studied the immunogenic effects of BSA-tuftsin conjugates _in vivo_. These were injected directly into mice and the immune response assayed by measuring the production of anti-BSA antibodies by a radioimmunoassay. We immunized mice intravenously with conjugates ranging form 25 to 200 µg protein. We observed that at 25 and 50 µg, BSA-tuftsin conjugates elicited, 8 days following injection, high titers of antibodies (IgG), whereas at the same doses the injection of BSA alone or BSA admixed with tuftsin evoked no measurable antibody titer.

Similar results were obtained with another conjugate of tuftsin-protein (Ribonuclease A;RNAse). Injection of 50 µg tuftsin-RNAse conjugate, intravenously, in aqeous solution, evoked a significant titer of anit-RNAse antibodies.

_Antigens for potential vaccination via tuftsin-conjugates_

In principle, any antigen may serve as a potent immunogen when applied covalently conjugated to tuftsin. At least 2 analogs of tuftsin, namely, $[Ala]^1$-tuftsin and tuftsin-Gly, when conjugated to a suitable antigen, are adapted to evoke an immune response, higher than that obtained by conjugates of tuftsin itself. Furthermore, tuftsin can also be attached to a synthetic polypeptide carrier, giving analogous results.

We prepared various polylysine and lysine-amino acid copolymers conjugated to tuftsin and these have similar properties. Tuftsin or its analogs can be conjugated also to hormones, sugars, oligosugars, nucleic acids, cell surfaces, membranes, vitamins, inorganic substances such as silica, glass, and to high synthetic polymers.

TABLE 1. Conjugation of tuftsin to antigen augments the immunogenic capacity of antigen-fed macrophages (*in vitro*):

| Antigen fed to macrophages | $^3H$-thymidine uptake (cpm) of lymph node cells in the presence and absence of | | | |
|---|---|---|---|---|
| | 0.5 μg KLH | -KLH | 100 μg BSA | -BSA |
| KLH | 12086 | 1741 | | |
| KLH + tuftsin | 38881 | 2163 | | |
| KLH-tuftsin-OH | 50457 | 2250 | | |
| H-tuftsin-KLH | 27902 | 1602 | | |
| BSA | | | 2715 | 337 |
| BSA + tuftsin | | | 31108 | 3650 |
| BSA -tuftsin-OH | | | 17394 | 1337 |
| H-tuftsin-BSA | | | 5198 | 919 |

Macrophages were pulsed with protein (10 μg/ml KLH or 100 μg/ml BSA), protein plus tuftsin or protein conjugated to tuftsin (via the N or C terminals), then overlaid with spleen lymphocytes. The latter, following 18 h of sensitization, were injected to mice, and the recruited effector T-cells were tested for antigen-specific T-cell proliferation 6 days later. The specific response to the test antigen was detected by $^3H$ thymidine incorporation, expressed in cpm.

TABLE 2. Augmentation of antibody production following injection of protein-tuftsin conjugates (*in vivo*):

A:

| Protein injected | Titer of antibodies produced ($^{125}I$ cpm) following injection of 25μg | 50 μg |
|---|---|---|
| BSA (control) | 0 | 0 |
| BSA + tuftsin (control) | 0 | 1581 |
| H-tuftsin-BSA (conjugate) | 18272 | 33227 |
| BSA-tuftsin-OH (conjugate) | 19360 | 37563 |

Mice were injected intravenously with 25 or 50 μg of protein in PBS. Eight days later, the mice were bled and the anti-BSA antibody titer was measured by radioimmunoassays. Results are expressed in cpm of $^{125}I$-rabbit antimouse IgG bound.

B:

| Protein injected | Titer of antibodies produced ($^{125}I$ cpm) |
|---|---|
| RNAse (control) | 84 |
| RNAse + tuftsin (control | 0 |
| H-tuftsin-RNAse (conjugate) | 6201 |

Mice were injected i.v. with 50 μg of protein in PBS. Eight days later the mice were bled and anti-RNA antibody titer was measured (at 1:10 dilution) by radioimmunoassays. Results are expressed in cpm of $^{125}I$-affinity purified goat anti-mouse Ig.

CLAIMS:

1. A composition of matter having highly potentiated antigenic activity which comprises a conjugate of tuftsin, or an active analog of tuftsin or of a short peptide containing the tuftsin sequence or of a short peptide containing the sequence of an active analog of tuftsin, with an antigenic moiety.
2. A composition according to claim 1, wherein the pentapeptide is tuftsin-Gly and the tetrapeptide is $[Ala]^1$-tuftsin.
3. A composition according to claim 1 or 2, wherein the antigenic moiety is an antigenic protein.
4. A composition according to any of claims 1 to 3, wherein the antigen is a viral, a bacterial or another pathogenic antigen.
5. A composition according to claim 1 or 2, wherein tuftsin or an analog thereof is conjugated to a natural or synthetic polypeptide, to a peptide hormone, to a non-peptide hormone, to a nucleic acid, or to any other antigenic hapten.
6. A composition of matter adapted to highly potentiate the immunogenic effect of an antigen, comprising such antigen chemically bonded to tuftsin or to an analog of tuftsin.
7. A composition according to claim 6, where the antigen is a weak immunogen, the activity of which is potentiated to a high degree by bonding to tuftsin or to an analog of same.
8. A composition of high immunogenic activity, for administration for the vaccination of mammals, comprising as active ingredient a composition according to any of claims 1 to 7, in a suitable injectable medium.

9. An antiviral, antibacterial or antiparasitic vaccine comprising a conjugate claimed in any of claims 1 to 7.

10. A vaccine having a highly immunogenic effect comprising a conjugate of tuftsin or an analog thereof with an antigen, substantially as hereinbefore described and with reference to the Examples.